# EUROPÄISCHE PATENTSCHRIFT

(11) **EP 3 836 001 B1**
(45) Veröffentlichungstag und Bekanntmachung des Hinweises auf die Patenterteilung: **31.08.2022**
(21) Anmeldenummer: 20210927.8
(22) Anmeldetag: 01.12.2020
(51) Int. Cl.: G06K 7/10, G06K 7/14

(54) **LESEN EINER VIELZAHL VON CODES**
READING OF A PLURALITY OF CODES
LECTURE D'UNE PLURALITÉ DE CODES

(30) Priorität: 12.12.2019 DE 102019134064
(43) Veröffentlichungstag der Anmeldung: 16.06.2021
(73) Patentinhaber: SICK AG, 79183 Waldkirch (DE)
(72) Erfinder: Hafner, Carl, 79276 Reute (DE); Maier, Rainer, 79271 St. Peter (DE); Heuser, Karsten, 79111 Freiburg (DE); Held, Matthias, 77933 Lahr (DE); Lais, Stefan, 79263 Simonswald (DE); Schlegel, Helge, 79211 Denzlingen (DE)

(56) Entgegenhaltungen:
- EP-A1- 3 040 906
- US-A1- 2013 020 391

## Beschreibung

Die Erfindung betrifft ein System mit einer Codelesevorrichtung und ein Verfahren zum parallelen Masselesen einer Vielzahl von Codes.

Gemäß der EU-Fälschungsschutzrichtlinie 2011/62/EU und der delegierten Verordnung (EU) 2016/161 müssen alle verschreibungspflichtigen Pharmaeinzelverpackungen von der Produktion bis zum Endkunden überwacht und mit einer EU-Datenbank abgeglichen werden, um mögliche Fälschungen zu identifizieren. Ähnliche Anforderungen sind auch in der Tabakrichtlinie 2014/40/EU zu finden.

Die einzelnen Verpackungen sind daher mit einem individuellen Code versehen, der eine eindeutige Seriennummer enthält. Bei jeder Übergabe müssen diese Codes gelesen werden, um mit Hilfe der Datenbank die korrekte Herkunft zu prüfen und so die Verifikationskette aufrecht zu erhalten.

Die DE 20 2019 104 044 U1 beschreibt ein System zum Verifizieren und Ausmelden von Medikamentenverpackungen anhand verpackungsspezifischer, individueller Seriennummern. Dabei geht es darum, wie die Kommunikation der Codeinformationen beispielsweise zwischen einer Klinikapotheke und einem Drittanbieter einer Verifikationsdatenbank erfolgen kann. Das eigentliche Erfassen der Codes, also das Codelesen, wird nicht näher betrachtet.

Die WO 2019/149908 A1 beschreibt ein Verfahren zum Überwachen der Lieferkette eines Produkts. Dazu werden verschiedene Verschlüsselungsverfahren einschließlich einer Blockchain vorgeschlagen. Dieser Ansatz nutzt die genannte EU-Datenbank nicht.

Um bei einer Übergabe sämtliche Produkte prüfen zu können, ist eine Scanlösung für die Codes erforderlich, die eine Leserate von 100 % garantiert. Das betrifft beispielsweise Großhändler verschreibungspflichtiger Pharmaprodukte, für die besagte EU-Vorschriften zwingend einzuhalten sind. Diese Problematik tritt jedoch verallgemeinert bei allen serialisierten Scanvorgängen auf, sowohl bei optischen Codes als auch beispielsweise bei RFID.

Die konkrete Herausforderung besteht darin, eine Vielzahl von Codes beispielsweise einer Kiste voller Medikamentenpackungen vollständig zu lesen. Eine Möglichkeit besteht darin, die einzelnen Verpackungen beziehungsweise deren Codes nacheinander mit einem Handscanner zu lesen. Das ist eine recht langwierige und mühsame Arbeit. Wenn nun außerdem nach Abschluss des Scanvorgangs nicht alle Codes gelesen sind, so erhält der Bediener keinerlei Ortsinformationen, bei welcher Verpackung der Scanvorgang nicht erfolgreich war. Es bleibt daher nichts Anderes übrig, als den gesamten Scanvorgang zu wiederholen, bis die Anzahl gelesener Codes der Anzahl der Verpackungen entspricht.

Ein anderer Ansatz besteht darin, einen kamerabasierten Codeleser zu verwenden, der ein Bild der Codes aufnimmt und diese somit nicht mehr seriell, sondern parallel liest. Das vereinfacht den Scanvorgang erheblich und eröffnet zudem die Möglichkeit, auf einer Anzeige darzustellen, welche Codes bereits gelesen wurden. Das System bietet dann aber keine zufriedenstellende Lösung an, wie mit den nicht gelesenen Codes umgegangen wird. Es wird nur die Möglichkeit angeboten, den Parallelleseprozess so lange zu wiederholen, bis es keine nicht gelesenen Codes mehr gibt. Viele Lesefehler werden sich jedoch reproduzieren lassen, so dass dies oft nicht zum Ziel führt. Als Alternative bleibt, Verpackungen mit nicht gelesenen Codes zu entfernen, bis die übrigen Codes vollständig gelesen werden. Für die verbleibenden Verpackungen ist dann eine individuelle Lösung zu suchen. Ein derartiges System wird beispielsweise von der Firma Strelen auf deren Internetseiten zum Stichwort Offline Codeprüfung angeboten.

Es sind Codelesesysteme bekannt, die eine Rückmeldung bezüglich nicht gelesener Codes geben. Beispielsweise projiziert ein Handscanner nach US 7 090 137 B1 verschiedene auf den Lesevorgang oder das Leseergebnis bezogene Informationen auf den Code oder in die Nähe des Codes. Damit könnte gegebenenfalls die oben beschriebene sequentielle Handlesung vereinfacht werden, indem Lesefehler sofort erkennbar werden, aber die mühsame Arbeit, sämtliche Verpackungen von Hand zu erfassen, entfällt dadurch nicht.

In der EP 3 040 906 A1 geht es um ein Handgerät zum Lesen komplexerer Etiketten mit mehreren Codes. Dabei wird eine Feedback-Information projiziert, welche Codes gelesen werden konnten, beispielsweise ein nicht gelesener Code mit einem "X" markiert, so dass der Benutzer diesen nicht gelesenen Code erneut scannen kann.

Aus der WO 2014/015058 A1 ist ein Lesetunnel bekannt, durch den nacheinander Pakete gefördert werden. Nach dem Lesebereich wird mit Licht in verschiedenen Farben angezeigt, beispielsweise rot und grün, ob auf dem Paket ein Code gelesen wurde oder nicht. Die Anzeige erfolgt mit Hilfe von seitlichen LEDs oder einer Projektion auf das Paket. Ein solcher Lesetunnel, der Pakete nacheinander verarbeitet, ist für die Verifikation einer größeren Menge von Verpackungen im Vergleich zu einem massiven Parallellesen langsam und umständlich.

Die US 2013/0020391 A1 offenbart ein automatisches Codelesesystem, das Objekte durch einen Lesebereich fördert und dort deren Codes liest. Vorab wird die Geometrie der Objekte vermessen, und diese Informationen werden genutzt, um die gelesenen Codeinhalte den Objekten zuzuordnen. Es werden Ausnahmesituationen erkannt, insbesondere dass auf einem Objekt kein Code gelesen werden konnte, und eine mögliche Auflösung ist, dass eine Bedienperson den Code mit einem Codescanner einscannt. Dazu kann der Bedienperson ein Bild des Lesebereichs während des vorangegangenen automatischen Leseversuchs mit Hervorhebung des von der Ausnahmesituation betroffenen Objekts angezeigt werden.

Es ist daher Aufgabe der Erfindung, das parallele Lesen einer Vielzahl von Codes zu verbessern.

Diese Aufgabe wird durch ein System mit einer Codelesevorrichtung und ein Verfahren zum parallelen Masselesen einer Vielzahl von Codes nach Anspruch 1 beziehungsweise 13 gelöst. Die Codelesevorrichtung ist für eine parallele Masselesung auch größerer Gruppen von Objekten mit Codes eingerichtet. Dazu ist eine Kameraeinheit mit mindestens einem Kamerakopf vorgesehen, die ein Bild der Objekte samt Codes aufnimmt. Eine Steuer- und Auswertungseinheit identifiziert die Codebereiche in dem Bild und liest die Codes. Eine Anzeigeeinheit stellt das Bild dar und markiert die gelesenen Codes beziehungsweise Objekte, sei es direkt beispielsweise durch eine farbliche Hervorhebung, Umrandung oder dergleichen, oder indirekt beispielsweise über die Angabe von Koordinaten. Vorzugsweise wird zudem die Anzahl der gelesenen Codes angezeigt.

Die Erfindung geht von dem Grundgedanken aus, der Codelesevorrichtung eine zum System gehöriges Handleseeinheit zum Lesen von Codes hinzuzufügen, beispielsweise einen Handscanner. Die Handleseeinheit ist Teil der Codelesevorrichtung, indem sie physisch daran angeschlossen ist oder jedenfalls in einer drahtlosen Kommunikationsverbindung zu deren Steuer- und Auswertungseinheit steht. Mittels der Handleseeinheit werden diejenigen Codes nachgelesen, die aus dem Bild der Kameraeinheit nicht gelesen werden konnten. Die entsprechende nachgelesene Codeinformation wird an die Steuer- und Auswertungseinheit übertragen, die somit die gelesenen Codes sowohl der Kameraeinheit als auch der Handleseeinheit sammeln, untereinander vergleichen und zählen kann.

Die Erfindung hat den Vorteil, dass durch das händische Nachlesen auf besonders einfache und komfortable Weise eine Leserate von 100 % erreicht werden kann. Da die Handleseeinheit in die Codevorrichtung integriert ist, steht sie immer sofort zur Verfügung, und es sind keine besonderen Schritte erforderlich, um deren Leseergebnisse mit den Leseergebnissen des parallelen Leseprozesses zu vereinen. Im Stand der Technik ist der Umgang mit nicht gelesenen Codes innerhalb des Systems gar nicht oder jedenfalls nur sehr umständlich möglich, wie einleitend beschrieben. Entweder wird dort der gesamte Lesevorgang wiederholt, was aufwändig ist und in vielen Fällen nur zum selben Fehler führt wie zuvor, oder die Objekte mit nicht gelesenen Codes müssen separiert und mit einer gesonderten Lösung behandelt werden.

Die Codelesevorrichtung weist vorzugsweise mehrere Kameraköpfe auf, wobei die Steuer- und Auswertungseinheit dafür ausgebildet ist, die Bilddaten der Kameraköpfe zu einem gemeinsamen Bild zusammenzufügen und/oder die Codes in den jeweiligen Bilddaten zu lesen und miteinander zu vergleichen. Die mehreren Kameraköpfe dienen dazu, ein größeres und/oder höher aufgelöstes Bild aufzunehmen. Die einzelnen Bilder der Kameraköpfe können zu einem Bild fusioniert werden, das dann behandelt wird wie das Bild nur eines einzigen Kamerakopfes, oder es werden jeweils Codes in den einzelnen Bildern gelesen, wobei die Auswertung der Bilder auch parallelisiert werden kann. Mit anderen Worten können die Detektionsergebnisse auf der Ebene von Bilddaten oder spätestens auf der Ebene von Codeinhalten zusammengefasst werden. Vorzugsweise ist der Überlapp der Sichtfelder der Kameraköpfe größer als ein Code, denn dann kann jeder Code in einem Bildbereich derselben Kamera gelesen werden und muss keine Übergangsbereiche berücksichtigen (Stitching). Dopplungen durch Lesen aus den Bildern mehrere Kameraköpfe werden wegen der eindeutigen Seriennummer der Codes erkannt.

Die Objekte befinden sich in einer Beförderungseinheit, beispielsweise in einer Kiste, auf einer Palette oder dergleichen. Die Kameraeinheit erfasst diese Beförderungseinheit von der relevanten Seite her, wo die Codes erkennbar sind, insbesondere aus der Draufsicht. Dazu wird beispielsweise die Kiste so gepackt, dass die einen Code tragenden Deckseiten der Objekte nach oben zeigen.

Die Objekte sind bevorzugt Verpackungen mit je einem eindeutigen Code, insbesondere Verpackungen für Medikamente oder Zigaretten. Die Pharma- und Zigarettenindustrie sind Zweige, in denen aufgrund von EU-Recht serialisierte Codes eine entscheidende Rolle spielen. Die Codes enthalten beispielsweise die Seriennummer sowie sonstige erforderliche Informationen für die Verifikation in der zentralen EU-Datenbank.

Die Steuer- und Auswertungseinheit ist bevorzugt dafür ausgebildet, die Objekte jeweils als Rechteck aus erfassten Kanten zu erkennen und insbesondere zu zählen. In vielen realen Anwendungen, so auch bei Verpackungen für Arzneien oder Zigarettenschachteln, ist die Geometrie der Objekte sehr einfach, und das kann genutzt werden, um in der Bildauswertung eine einfache Segmentierung umzusetzen. Quaderförmige Verpackungen sind im Bild aus der Perspektive der Kameraeinheit nebeneinanderliegende Rechtecke. Somit kann eine Kantendetektion im Bild und eine Zusammenfassung zu Rechtecken die Objekte trennen und lokalisieren. Das hat mehrere Vorteile. Zum einen kann die Steuer- und Auswertungseinheit auf diese Weise die Gesamtzahl der erfassten Objekte selbst bestimmen, die dann mit der Anzahl gelesener Codes verglichen werden kann, um festzustellen, ob alle Codes gelesen wurden beziehungsweise wie viele Codes mit der Handleseeinrichtung nachgelesen werden müssen. Zum anderen können dann die ganzen Objekte markiert werden, deren Codes bereits gelesen beziehungsweise noch nicht gelesen sind, und dies wiederum vereinfacht die Erkennung der noch mit der Handleseeinheit nachzulesenden Codes. Prinzipiell ist alternativ auch eine komplexe Segmentierung ohne Vorwissen über die Geometrie der Objekte denkbar, aber dann ist es erheblich schwieriger, mit der erforderlichen Verlässlichkeit wirklich alle Objekte zu trennen. Ein Segmentierungsfehler würde fälschlich zur Annahme einer Leserate von 100 % führen.

Die Steuer- und Auswertungseinheit ist dafür ausgebildet zu prüfen, ob ein nachgelesener Code bereits zuvor gelesen wurde. Wenn die Handleseeinheit den Codeinhalt eines weiteren gelesenen Codes übermittelt, bedeutet das keineswegs automatisch, dass sich die Gesamtzahl gelesener Code erhöht. Der Bediener könnte einen bereite über die Kameraeinheit gelesenen Code oder einen zuvor schon nachgelesenen Code erfasst haben. Dies wird über die eindeutige Codeinformation geprüft und erkannt, so dass von der Handleseeinheit übertragene Codeinformationen zu schon bekannten Codes ignoriert werden können und nur neue Codeinformationen hinzugefügt werden beziehungsweise die Anzahl gelesener Codes dementsprechend heraufgezählt wird. Eine entsprechende Prüfung auf bereits bekannte Codes finden im Übrigen vorzugsweise auch für die Parallellesung über die Kameraeinheit statt, sei es um Mehrfachaufnahmen von mehreren Kameraköpfen, wiederholten Aufnahmen oder mehrerer redundant auf einem Objekt angebrachter Codes aufzufangen.

Die Steuer- und Auswertungseinheit ist bevorzugt dafür ausgebildet, Codes und/oder Objekte zu markieren, zu denen noch kein Code gelesen wurde. Wenn die Objekte dicht gepackt sind, wie bei Arzneimittelverpackungen oder Zigarettenschachteln in einem Transportbehältnis, sind die nicht gelesenen Codes bereits deshalb recht gut erkennbar, weil dort die Markierung für einen gelesenen Code fehlt. Dennoch kann es hilfreich sein, explizit eine Markierung der nicht gelesenen Codes zu ergänzen. Beispielsweise werden unterschiedliche Farben genutzt, etwa die gelesenen Codes beziehungsweise zugehörigen Objekte grün und die nicht gelesenen Codes beziehungsweise zugehörigen Objekte rot markiert.

Die Steuer- und Auswertungseinheit ist bevorzugt dafür ausgebildet, nachgelesene Codes zu lokalisieren. Die Handleseeinheit liest den Code dort, wohin der Bediener sie richtet. Ohne weitere Maßnahmen erfährt dann die Steuer- und Auswertungseinheit nur die Codeinformation, jedoch nicht, zu welchem Objekt beziehungsweise Ort im Bild diese Codeinformation gehört. Nach dieser vorteilhaften Ausführungsform wird der nachgelesene Code zusätzlich einem bestimmten Objekt oder Ort im Bild zugeordnet. Das kann durch eine händische Eingabe, eine Eigenlokalisation durch Sensorik oder dergleichen der Handleseeinheit oder durch zusätzliche Bildauswertung erfolgen.

Die Steuer- und Auswertungseinheit ist bevorzugt dafür ausgebildet, einen von der Handleseeinheit auf den Objekten erzeugten Leselichtfleck zu lokalisieren. Das ist eine besonders einfache Umsetzungsmöglichkeit, nachgelesene Codes zu lokalisieren. Wenn die Steuer- und Auswertungseinheit in engem zeitlichen Zusammenhang einen Leselichtfleck in dem Bild erkennt und von der Handleseeinheit die Codeinformation zu einem gelesenen Code erhält, dann wird diese Codeinformation mit dem Code oder Objekt mit dem Leselichtfleck verknüpft.

Die Steuer- und Auswertungseinheit ist bevorzugt dafür ausgebildet, einen nachgelesenen Code zu markieren. Damit erhält der Bediener eine Rückmeldung, dass dieser Code nun nachgelesen ist. Beispielsweise wechselt im Bild an dem nachgelesenen Code oder zugehörigen Objekt die Markierung, etwa von rot zu grün, oder der nachgelesene Code wird vorübergehend mit einer gesonderten, unterscheidbaren Markierung hervorgehoben, etwa durch eine weitere Farbe, eine besonders helle oder dicke Markierung oder dergleichen.

Die Anzeigeeinheit ist bevorzugt als Touchscreen ausgebildet, über den die Codelesevorrichtung bedient wird. Die Anzeigeeinheit kann dadurch zugleich die Funktion einer Bedieneinheit erfüllen (HMI, Human Machine Interface), wobei zusätzliche Bedienelemente möglich bleiben. Der Touchscreen kann auch für eine alternative Lokalisierung nachgelesener Codes genutzt werden: Der Bediener berührt einen Bereich der Anzeige, um einen soeben nachgelesenen oder sogleich nachzulesenden Code mit diesem Ort zu verknüpfen.

Die Kameraeinheit weist bevorzugt eine veränderbare Beleuchtungseinheit auf, insbesondere mit mehreren Beleuchtungsmodulen, wobei die Steuer- und Auswertungseinheit dafür ausgebildet ist, für das parallele Lesen der Codes die Objekte mehrfach unter verschiedenen Beleuchtungen aufzunehmen, insbesondere bei Beleuchtung aus unterschiedlichen Richtungen. Die Beleuchtungseinheit ist in der Lage, unterschiedliche Beleuchtungsszenarien zu erzeugen, unter denen womöglich andere beziehungsweise mehr Codes gelesen werden können. Dazu wird insbesondere die Beleuchtungsstärke und/oder die Richtung der Beleuchtung variiert. Eine bevorzugte Ausführungsform sieht vor, je ein Beleuchtungsmodul rechts und links der Kameraeinheit zu positionieren und drei Aufnahmen bei Beleuchtung von je einer beziehungsweise von beiden Seiten zu erzeugen. So werden nachteilige Effekte durch Eigenblendung sowie Unter- und Übersteuerung kompensiert. Die mehreren Bilder werden je nach Ausführungsform zunächst zu einem Bild besserer Qualität fusioniert, ähnlich einer HDR-Aufnahme (High Dynamic Range), oder die Steuer- und Auswertungseinheit liest Codes nacheinander aus mehreren oder jedem der Bilder, wobei Dopplungen anhand der Seriennummer aussortiert werden können.

Die Steuer- und Auswertungseinheit ist vorzugsweise für einen Aggregationsmodus ausgebildet, in dem zunächst ein Aggregationscode gelesen wird und die danach gelesenen Codes diesem Aggregationscode zugeordnet werden. Der Aggregationscode wird vorzugsweise über die Datenbank beziehungsweise dessen Betreiber bereitgestellt, über die beziehungsweise den die Verifikation erfolgt. Unter dem Aggregationscode kann eine Vielzahl von verifizierten Codes zusammengefasst werden. Beispielsweise wird ein solcher Aggregationscode auf einer Kiste, Palette oder sonstigen Beförderungseinheit angebracht, die dann vorzugsweise geschlossen oder versiegelt wird. Solange die Objekte mit den unter dem Aggregationscode zusammengefassten Codes beieinanderbleiben, genügt es, jeweils nur den Aggregationscode zu lesen und zu verifizieren, unter dem alle zugeordneten Codes in der Datenbank als zusammengehörig gefunden werden.

Die Codelesevorrichtung weist bevorzugt eine Schnittstelle zu einem übergeordneten System auf, in dem die gelesenen Codes verifiziert und/oder ausgebucht werden. Das übergeordnete System ist vorzugsweise ein Netzwerk oder eine Cloud mit einer zentralen Datenbank. Diese Datenbank wird häufig von einem Drittanbieter betrieben, der die dorthin übertragenen Codes bei Bedarf verifiziert und ausbucht. Dabei bedeutet Verifizieren, dass die Authentizität des Codes bestätigt wird, um Fälschungen auszuschließen, während beim Ausbuchen die Übergabe an den Endkunden erfolgt ist, wo das Objekt, beispielsweise ein Medikament, verbraucht und jedenfalls nicht weiter überwacht wird. Für weitere Details hierzu wird auch nochmals auf die einleitend genannte DE 20 2019 104 044 U1 verwiesen.

Das erfindungsgemäße Verfahren kann auf ähnliche Weise weitergebildet werden und zeigt dabei ähnliche Vorteile. Derartige vorteilhafte Merkmale sind beispielhaft, aber nicht abschließend in den sich an die unabhängigen Ansprüche anschließenden Unteransprüchen beschrieben.

Die Erfindung wird nachstehend auch hinsichtlich weiterer Merkmale und Vorteile beispielhaft anhand von Ausführungsformen und unter Bezug auf die beigefügte Zeichnung näher erläutert. Die Abbildungen der Zeichnung zeigen in:
- Fig. 1: eine Übersichtsdarstellung einer Codelesevorrichtung mit Kameraeinheit zum parallelen Lesen von Codes und Handleseeinheit zum Nachlesen von Code;
- Fig. 2: ein beispielhaftes Kamerabild einer Vielzahl von Objekten mit Codes und Markierung der mittels der Kameraeinheit gelesenen Codes; und
- Fig. 3: das Kamerabild gemäß Figur 2 nun zusätzlich mit gesonderter Markierung der mittels der Kameraeinheit nicht lesbaren Codes.

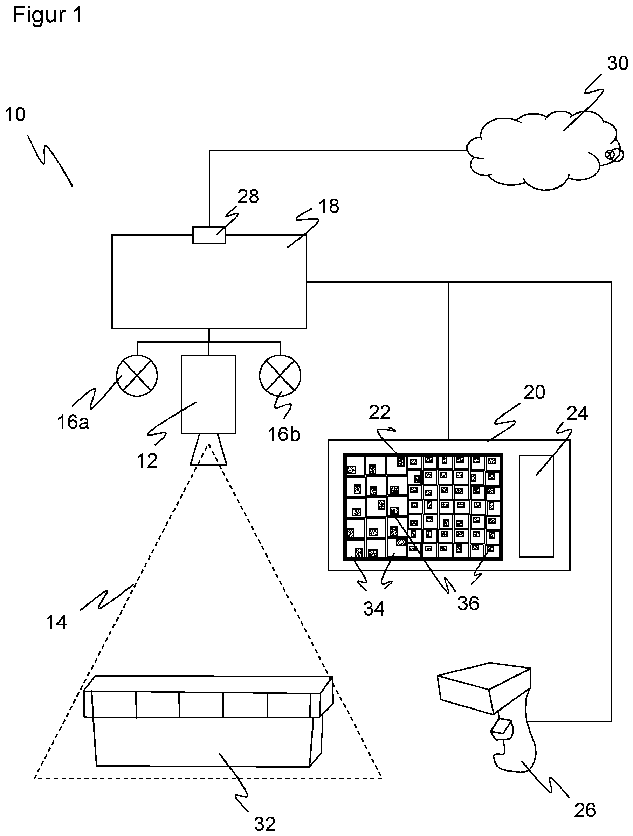

Figur 1 zeigt eine Übersichtsdarstellung einer Codelesevorrichtung 10. Sie ist als Komplettsystem ausgebildet, das einen kamerabasierten parallelen Leseprozess anbietet, um eine Vielzahl von Codes zu lesen, und zudem die Möglichkeit vorsieht, innerhalb des Komplettsystems etwaige von dem parallelen Leseprozess nicht gelesene Codes nachzulesen und so eine Leserate von 100 % zu erreichen.

Die Codelesevorrichtung 10 weist eine Kameraeinheit 12 auf, deren Kamerakopf in Figur 1 nur symbolisch gezeigt ist. Die Kameraeinheit 12 umfasst ohne gesonderte Darstellung einen vorzugsweise hochauflösenden Bildsensor, eine geeignete Optik und eine Schnittstelle zumindest zur Ausgabe der jeweils aufgenommenen Bilddaten. Falls das Sichtfeld 14 eines einzelnen Kamerakopfes nicht ausreicht, können mehrere Kameraköpfe nebeneinander angeordnet werden. Der Kameraeinheit 12 ist eine Beleuchtungseinheit zugeordnet, die in Figur 1 mit zwei Beleuchtungsmodulen 16a-b zu beiden Seiten ausgestaltet ist. Die Beleuchtungsmodule 16a-b sind vorzugsweise besonders lichtstark mit entsprechend leistungsfähigen LEDs oder Laserdioden.

Eine Steuer- und Auswertungseinheit 18 greift auf die Bilddaten der Kameraeinheit 12 zu, wertet sie aus und steuert während der Aufnahmen die Beleuchtungsmodule 16a-b. Auf einer Anzeige 20 können Bilder der Kameraeinheit 12 und Auswertungsergebnisse dargestellt werden. Die Anzeige 20 ist vorzugsweise als Touchscreen ausgebildet und dient dann zugleich als Bedieneinheit der Codelesevorrichtung 10. Beispielsweise ist auf der Anzeige 20 ein erster Bereich 22 zum Darstellen von Bildern und ein zweiter Bereich 24 für Bedienelemente vorgesehen.

Weiterhin ist an die Steuer- und Auswertungseinheit 18 drahtlos oder drahtgebunden ein Handscanner 26 angeschlossen. Über eine Schnittstelle 28 kommuniziert die Steuer- und Auswertungseinheit 18 mit einem übergeordneten System, insbesondere einer Cloud 30, und steht dadurch in Kontakt mit einer Datenbank in der Cloud 30. Die Schnittstelle 28 kann nach jedem bekannten Standard eingerichtet sein, beispielsweise drahtgebunden oder drahtlos, beispielsweise per WiFi oder Mobilfunk. Im Sichtfeld 14 der Kameraeinheit 12 ist eine Kiste 32 angeordnet, in der sich eine Vielzahl von Objekten 34 mit darauf angebrachten optischen Codes 36 befindet. Die Codes 36 sind serialisiert, sie sind folglich eindeutig identifizierbar. Zusatzinformationen bezüglich eines Verpackungsinhalts, einer Chargennummer, eines Herstell- oder Verfallsdatums, einer Lieferadresse und dergleichen sind auch möglich.

Die verschiedenen Elemente der Codelesevorrichtung 10 sind durch ein Gerüst mechanisch miteinander verbunden, so dass das Komplettsystem auch physisch eine Einheit bildet. An dem Gerüst sind Kameraeinheit 12 samt Beleuchtungsmodulen 16a-b derart befestigt, dass die Kiste 32 innerhalb des Tiefenschärfenbereichs aufgenommen wird, wenn sie auf eine am Gerüst vorgesehenen Auflage gestellt wird. Die Steuer- und Auswertungseinheit 18 kann im Gerüst verborgen sein, und darauf wird über die als Touchscreen ausgestaltete Anzeige 20 und/oder alternative Bedienelemente zugegriffen, die jeweils gut erreichbar am Gerüst angebracht sind. Für den Handscanner 26 ist vorzugsweise zumindest eine Ablagestation am Gerüst vorgesehen, die auch ein drahtloses Gerät auflädt, oder der Handscanner 26 kann durch eine Anschlussleitung oder eine Halterungsschnur verbunden sein, die verhindert, dass der Handscanner 26 von der Codelesevorrichtung 10 entfernt wird.

Um nun sämtliche Codes 36 in der Kiste 32 zu lesen, wird zunächst von der Kameraeinheit 12 ein Bild der Kiste mit den darin befindlichen Objekten 34 und Codes 36 aufgenommen, wie es beispielhaft im ersten Bereich 22 der Anzeige 20 dargestellt ist. Die Perspektive der Kameraeinheit 12, hier in der Draufsicht, sowie die Orientierung der Objekte 34 in der Kiste 32 sind aufeinander abgestimmt, so dass alle Codes 36 im Bild sichtbar sind.

Die Steuer- und Auswertungseinheit 18 findet mittels Bildauswertungsverfahren die Codebereiche mit den Codes 36 und liest deren Codeinhalte. Dabei sind alle ein- und zweidimensionalen Codestandards denkbar. Das kamerabasierte Lesen optischer Codes an sich ist bekannt und wird hier nicht näher beschrieben.

Im Falle mehrerer Kameraköpfe nimmt jeder Kamerakopf seinen Teilausschnitt des Sichtfeldes 14 auf. Diese Bilddaten werden dann fusioniert, und die Codes 36 werden in dem gemeinsamen Bild gelesen. Alternativ werden Codes 36 aus den einzelnen Bildern gelesen und die Daten dann erst auf Ebene von gelesenen Codeinformationen gesammelt.

In bevorzugten Ausführungsformen werden mehrere Aufnahmen erzeugt. Erneut ist alternativ denkbar, anschließend zunächst die Bilddaten zu fusionieren, um ein qualitativ höherwertiges Bild zu erhalten und darin die Codes 36 zu lesen, oder schon in den jeweiligen Bildern Codes 36 zu lesen, um so letztlich für jeden Code 36 mehrere Versuche auf unterschiedlichen Bilddaten vorzunehmen. Ein Beispiel für das Erfassen von höherwertigen Bilddaten durch Mehrfachaufnahme ist HDR (High Dynamic Range).

Solche Mehrfachaufnahmen werden vorzugsweise in verschiedenen Beleuchtungsszenarien erzeugt, indem unterschiedliche Beleuchtungsmodule 16a-b aktiviert und/oder deren Beleuchtungsintensität verändert wird. Ein Beispiel ist eine dreistufige Beleuchtungssequenz mit drei Aufnahmen, für die der zunächst das linke Beleuchtungsmodul 16a, dann das rechte Beleuchtungsmodul 16b und schließlich beide Beleuchtungsmodule 16a-b eingeschaltet werden. Dadurch werden Lesefehler durch störende Reflexionen minimiert. Die Anzahl der Beleuchtungsmodule 16a-b und damit die Anzahl möglicher Beleuchtungsszenarien sowie die Reihenfolge in der Beleuchtungssequenz ist nicht festgelegt. Vielmehr können die Beleuchtungsszenarien und Mehrfachaufnahmen anwendungsbezogen parametriert werden.

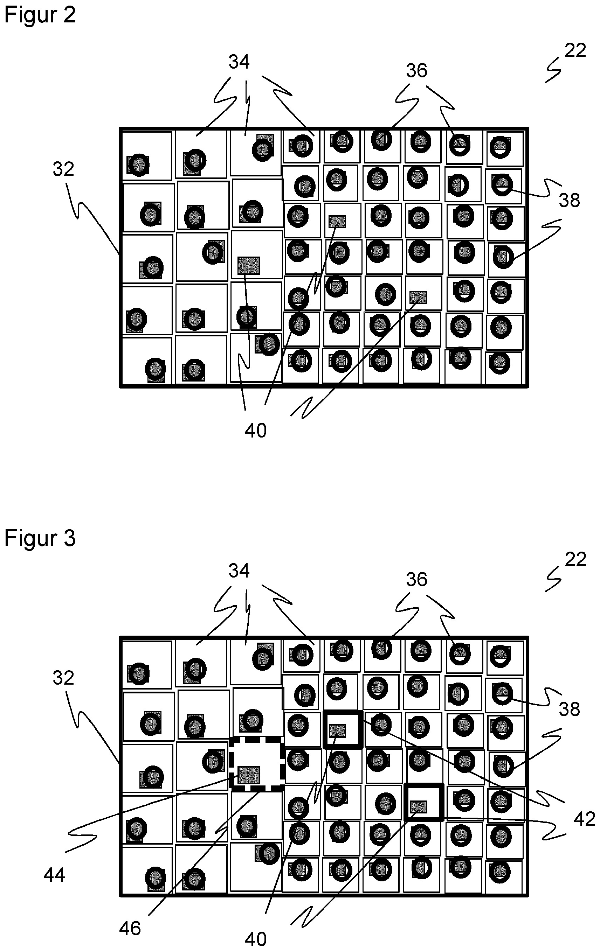

Durch den parallelen Leseprozess auf Basis der Bilddaten der Kameraeinheit 12 werden nur im Idealfall sämtliche Codes 36 gelesen. Um eine vollständige Leserate von 100 % zu erreichen, werden im Anschluss die bisher nicht lesbaren Codes 36 mit dem Handscanner 26 nachgelesen. Dabei wird der Bediener durch die Anzeige 20 unterstützt, auf der beispielsweise die bereits gelesenen Codes 36 oder die Orte von als Objekt 34 oder Code 36 erkannten Bereiche markiert werden, in denen noch nachgelesen werden muss. Außerdem wird vorzugsweise die Anzahl gelesener Codes 36 und, sofern diese Anzahl vorgegeben ist oder per Bildauswertung ermittelt wurde, auch die Anzahl insgesamt vorhandener Codes 36 angezeigt. Diese Möglichkeiten werden später unter Bezugnahme auf die Figuren 2 und 3 noch näher erläutert.

Über die Schnittstelle 28 werden die Codeinformationen in einer zentralen Datenbank, die hier beispielhaft in der Cloud 30 implementiert ist, verifiziert und bei Übergabe an den Endkunden auch ausgebucht. Die Datenbank, insbesondere eine Pharma- oder Tabak-Verifikationsplattform, wird in der Regel von einem Dienstleister betrieben. Die Steuer- und Auswertungseinheit 18 leistet sämtliche auf Seiten der Codelesevorrichtung 10 erforderlichen Schritte der Kommunikation. Die Datenbank und die konkrete Ausgestaltung der Kommunikation mit der Datenbank sind nicht Gegenstand der Erfindung, und dazu wird beispielsweise nochmals auf die einleitend genannte DE 20 2019 104 044 U1 sowie die Möglichkeiten der Absicherung durch Verschlüsselung und Blockchain nach WO 2019/149908 A1 verwiesen.

Figur 2 zeigt in vergrößerter Darstellung ein beispielhaftes Bild der Kiste 32, wie es dem Bediener im ersten Bereich 22 der Anzeige 20 präsentiert werden kann. Die im Parallelleseprozess auf Basis der Aufnahme der Kameraeinheit 12 gelesenen Codes 36 werden mit einer ersten Markierung 38 gekennzeichnet, hier rein beispielhaft mit einem Kringel insbesondere in grüner Farbe. Auf diese Weise kann der Bediener sofort die noch nicht gelesenen Codes 40 erkennen und diese gezielt mit dem Handscanner 26 nachlesen.

In dieser Ausführungsform ist die Bildauswertung darauf beschränkt, Codebereiche zu identifizieren und so viele Code 36 wie möglich zu lesen. Daher ist die Ortsinformation der im Parallelprozess gelesenen Codes 36 bekannt, um damit die ersten Markierungen 38 an den richtigen Stellen zu platzieren. Die Objekte 34 selbst wurden jedoch nicht segmentiert. Die nicht gelesenen Codes 40 wurden aber möglicherweise durchaus beispielsweise anhand ihres Kontrasts als Codes 36 erkannt, nur ist anschließend der Decoder daran gescheitert, die Codeinformation auszulesen. Daher ist in einer weiteren Ausführungsform denkbar, auch die nicht gelesenen Codes 40 mit einer zweitem Markierung zu versehen, beispielsweise in rot, um noch deutlicher hervorzuheben, wohin der Handscanner 26 noch geführt werden muss.

Figur 3 zeigt nochmals ein beispielhaftes Bild der Kiste 32, um einige weitere Ausführungsformen mit Anzeige zusätzlicher Informationen zur Unterstützung des Bedieners beim Nachlesen zu erläutern. In einer Variante werden durch Bildauswertung die Objekte 34 segmentiert. Das hat zwei Vorteile. Zum einen können als zweite Markierung 42 für nicht gelesene Codes 40 die zugehörigen Objekte 34 markiert werden, beispielsweise durch eine Umrandung, um die Sichtbarkeit zu verbessern. Diese zweite Markierung 42 ist auch dann möglich, wenn beim parallelen Leseversuch eines Codes 36 nicht einmal erkannt wurde, dass es sich überhaupt um einen Code 36 handelt. Denn nun ist das zugehörige Objekt 34 durch die Segmentierung identifiziert, das dann, wenn daran kein Code 36 gelesen wurde, zwangsläufig einen nicht gelesenen Code 40 tragen muss. Zum anderen können nun die Objekte 34 in der Kiste 32 gezählt werden, d.h. die zu erreichende Gesamtzahl gelesener Codes 36 ist ohne Vorwissen oder manuelles Zählen bekannt. Damit weiß die Steuer- und Auswertungseinheit 18, wann alle Codes 36 gelesen sind und nichts mehr nachgelesen werden muss.

Da es sich bei den Objekten 34 regelmäßig um quaderförmige Verpackungen womöglich auch zumindest grob bekannter Abmessungen handelt, ist hier keineswegs eine mächtige universelle Objektsegmentierung erforderlich, die aber an sich auch einsetzbar wäre. Vielmehr genügt eine vergleichsweise einfache Kantendetektion mit anschließender Zusammenführung von Rechtecken. Das verringert die Komplexität der Objektsegmentierung erheblich und verringert die Fehlerrate, denn gerade für eine automatische Bestimmung der Gesamtanzahl als Beurteilungsmaßstab für eine Leserate von 100 % sollten die Objekte 34 sehr zuverlässig segmentiert und gezählt werden.

Wenn der Bediener einen bestimmten bisher nicht gelesenen Code 44 nachliest, kann die Steuer- und Auswertungseinheit 18 anhand der eindeutigen Seriencodes erkennen, ob dieser Code 44 schon einmal gelesen wurde. Sie hat aber zunächst keine Möglichkeit, den nachgelesenen Code 44 innerhalb des Bildes zu lokalisieren, denn der Handscanner 26 liefert nur den Codeinhalt. Für den Bediener ist es jedoch eine erhebliche Erleichterung, wenn der nachgelesene Code 44 künftig als gelesen markiert ist oder eine besondere Markierung 46 erhält und diese dann vorzugsweise zu einer ersten Markierung 38 als gelesen wechselt.

Es gibt mehrere Möglichkeiten, wie diese Lokalisierung doch erreicht werden kann. Prinzipiell könnte der Handscanner 26 diese Information durch Eigenlokalisierung und Abstandsmessung mitliefern. Auch der Bediener könnte den Bezug selbst herstellen, indem beispielsweise der Bereich des gerade nachgelesenen Codes 44 beziehungsweise des im Anschluss nachgelesenen Codes 44 auf dem Touchscreen der Anzeige 20 berührt wird. Besonders bevorzugt wird der nachgelesene Code 44 automatisch durch Bildauswertung erkannt. Dazu wird der Leselichtfleck lokalisiert, den der Handscanner 26 auf dem nachgelesenen Code 44 erzeugt. Bei entsprechend engem Zeitbezug zwischen Lokalisieren des Leselichtflecks und Übermitteln eines Codeinhalts von dem Handscanner 26 verknüpft die Steuer- und Auswertungseinheit 18 diese beiden Ereignisse und erkennt so den Ort des nachgelesenen Codes 44 im Bild.

In einer bevorzugten Weiterbildung werden nicht immer ganze Gruppen von Codes 36 verifiziert, sondern eine Vielzahl von Codes 36 wird unter einem sogenannten Aggregationscode zusammengefasst und über diesen Aggregationscode mit der Datenbank abgeglichen. Beispielsweise wird ein Aggregationscode auf eine geschlossene Kiste 32 geklebt, nachdem sämtliche Codes 36 darin gelesen und verifiziert wurden. Der gesamte Inhalt der Kiste 32 kann nun mit erheblich verringertem Aufwand über den Aggregationscode erfasst werden. Der Aggregationscode wird von dem Datenbankbetreiber in der Cloud 30 zur Verfügung gestellt, wo auch später die Verknüpfung zwischen Aggregationscode zu den darunter zusammengefassten Codes 36 erfolgt.

Die Codevorrichtung 10 kann dafür einen Aggregationsmodus anbieten. Dabei wird zuerst, sei es mittels der Kameraeinheit 12 oder dem Handscanner 26, der Aggregationscode gelesen. Anschließend werden Codes 36 mit dem oben beschrieben Verfahren gelesen und auf den Aggregationscode aufgebucht.

## Patentansprüche

1. System mit einem Gerüst, das eine Auflage aufweist, auf die eine Beförderungseinheit (32) mit einer Vielzahl dicht gepackt nebeneinander angeordneter Objekte (34) gestellt ist, und mit einer Codelesevorrichtung (10) für eine parallele Masselesung einer Vielzahl von Codes (36) auf der Vielzahl dicht gepackt angeordneter Objekte (34), wobei die Codelesevorrichtung (10) eine an dem Gerüst befestigte Kameraeinheit (12) mit mindestens einem Kamerakopf zum Aufnahmen eines Bildes der Objekte (34), eine Steuer- und Auswertungseinheit (18), die dafür ausgebildet ist, in dem Bild Codebereiche der Codes (36) aufzufinden und die Codeinformation der Codes (36) zu lesen, sowie eine Anzeigeeinheit (20) aufweist, um das Bild darzustellen und die gelesenen Codes (36) und/oder Objekte (34) mit gelesenen Codes (36) zu markieren, wobei die Codelesevorrichtung (10) weiterhin eine Handleseeinheit (26) zum Lesen von Codes (36, 40) aufweist, um nicht gelesene Codes (40) nachzulesen, die aus dem Bild der Kameraeinheit (12) nicht gelesen werden konnten, und die nachgelesene Codeinformation an die Steuer- und Auswertungseinheit (18) zu übertragen, wobei die Steuer- und Auswertungseinheit (18) dafür ausgebildet ist zu prüfen, ob ein nachgelesener Code (36, 40) bereits zuvor gelesen wurde.

2. System nach Anspruch 1,
wobei die Codelesevorrichtung (10) mehrere Kameraköpfe aufweist, wobei die Steuer- und Auswertungseinheit (18) dafür ausgebildet ist, die Bilddaten der Kameraköpfe zu einem gemeinsamen Bild zusammenzufügen und/oder die Codes (36) in den jeweiligen Bilddaten zu lesen und miteinander zu vergleichen.

3. System nach Anspruch 1 oder 2,
wobei die Objekte (34) Verpackungen mit je einem eindeutigen Code (36) sind, insbesondere Verpackungen für Medikamente oder Zigaretten.

4. System nach einem der vorhergehenden Ansprüche,
wobei die Steuer- und Auswertungseinheit (18) dafür ausgebildet ist, die Objekte (34) jeweils als Rechteck aus erfassten Kanten zu erkennen und insbesondere zu zählen.

5. System nach einem der vorhergehenden Ansprüche,
wobei die Steuer- und Auswertungseinheit (18) dafür ausgebildet ist, Codes (36) und/oder Objekte (34) zu markieren, zu denen noch kein Code (36, 40) gelesen wurde.

6. System nach einem der vorhergehenden Ansprüche,
wobei die Steuer- und Auswertungseinheit (18) dafür ausgebildet ist, nachgelesene Codes (40, 44) zu lokalisieren.

7. System nach einem der vorhergehenden Ansprüche,
wobei die Steuer- und Auswertungseinheit (18) dafür ausgebildet ist, einen von der Handleseeinheit (26) auf den Objekten (34) erzeugten Leselichtfleck zu lokalisieren.

8. System nach einem der vorhergehenden Ansprüche,
wobei die Steuer- und Auswertungseinheit (18) dafür ausgebildet ist, einen nachgelesenen Code (40, 44) zu markieren.

9. System nach einem der vorhergehenden Ansprüche,
wobei die Anzeigeeinheit (20) als Touchscreen ausgebildet ist, über den die Codelesevorrichtung (10) bedient wird.

10. System nach einem der vorhergehenden Ansprüche,
wobei die Kameraeinheit (12) eine veränderbare Beleuchtungseinheit (16a-b) aufweist, insbesondere mit mehreren Beleuchtungsmodulen (16a-b), und wobei die Steuer- und Auswertungseinheit (18) dafür ausgebildet ist, für das parallele Lesen der Codes (36) die Objekte (34) mehrfach unter verschiedenen Beleuchtungen aufzunehmen, insbesondere bei Beleuchtung aus unterschiedlichen Richtungen.

11. System nach einem der vorhergehenden Ansprüche,
wobei die Steuer- und Auswertungseinheit (18) für einen Aggregationsmodus ausgebildet ist, in dem zunächst ein Aggregationscode gelesen wird und die danach gelesenen Codes (36) diesem Aggregationscode zugeordnet werden.

12. System nach einem der vorhergehenden Ansprüche,
die eine Schnittstelle (28) zu einem übergeordneten System (30) aufweist, in dem die gelesenen Codes (36) verifiziert und/oder ausgebucht werden.

13. Verfahren zum parallelen Masselesen einer Vielzahl von Codes (36) auf einer Vielzahl dicht gepackt nebeneinander in einer Beförderungseinheit (32) angeordneter Objekte (34), bei dem mit einer an einem Gerüst befestigten Kameraeinheit (12) ein Bild der Objekte (34) aufgenommen wird, in dem Codebereiche der Codes (36) aufgefunden werden und die Codeinformation der Codes (36) gelesen wird und wobei das Bild dargestellt wird und die gelesenen Codes (36) und/oder Objekte (34) mit gelesenen Codes (36) markiert werden, wobei die Beförderungseinheit (32) auf eine am Gerüst vorgesehene Auflage gestellt wird und mit einer Handleseeinheit (26) zum Lesen von Codes (36, 40) nicht gelesene Codes (40) nachgelesen werden, die aus dem Bild der Kameraeinheit (12) nicht gelesen werden konnten, und die von der Kameraeinheit (12) und der Handleseeinheit (26) gelesenen Codeinformationen gemeinsam gesammelt werden, wobei geprüft wird, ob ein nachgelesener Code (36, 40) bereits zuvor gelesen wurde.

## Claims

1. A system having a frame comprising a support on which a transport unit (32) with a plurality of closely packed objects (34) arranged side by side is placed, and having a code reading device (10) for parallel mass reading of a plurality of codes (36) on the plurality of closely packed objects (34), wherein the code reading device (10) comprises a camera unit (12) fixed to the frame and having at least one camera head for taking an image of the objects (34), a control and evaluation unit (18) configured to find code areas of the codes (36) in the image and to read the code information of the codes (36) and a display unit (20) for displaying the image and marking the read codes (36) and/or objects (34) with read codes (36), wherein the code reading device (10) further comprises a hand-held reading unit (26) for reading codes (36, 40) in order to re-read unread codes (40) that could not be read from the image of the camera unit (12), and to transmit the re-read code information to the control and evaluation unit (18), the control and evaluation unit (18) being configured to check whether a re-read code (36, 40) has already been read before.

2. The system according to claim 1,
wherein the code reading device (10) comprises a plurality of camera heads, wherein the control and evaluation unit (18) is configured to combine the image data of the camera heads into a common image and/or to read the codes (36) in the respective image data and to compare them with each other.

3. The system according to claim 1 or 2,
wherein the objects (34) are packages each having a unique code (36), in particular packages for medicines or cigarettes.

4. The system according to any of the preceding claims,
wherein the control and evaluation unit (18) is configured to recognize and in particular count the objects (34) as a respective rectangle of detected edges.

5. The system according to any of the preceding claims,
wherein the control and evaluation unit (18) is configured to mark codes (36) and/or objects (34) for which no code (36, 40) has yet been read.

6. The system according to any of the preceding claims,
wherein the control and evaluation unit (18) is configured to locate re-read codes (40, 44).

7. The system according to any of the preceding claims,
wherein the control and evaluation unit (18) is configured to locate a reading light spot generated by the hand-held reading unit (26) on the objects (34).

8. The system according to any of the preceding claims,
wherein the control and evaluation unit (18) is configured to mark a re-read code (40, 44).

9. The system according to any of the preceding claims,
wherein the display unit (20) is configured as a touch screen via which the code reading device (10) is operated.

10. The system according to any of the preceding claims,
wherein the camera unit (12) has a variable illumination unit (16a-b), in particular having a plurality of illumination modules (16a-b), and wherein the control and evaluation unit (18) is configured to record the objects (34) a plurality of times under different illuminations for the parallel reading of the codes (36), in particular with illumination from different directions.

11. The system according to any of the preceding claims,
wherein the control and evaluation unit (18) is configured for an aggregation mode in which an aggregation code is first read and the codes (36) read thereafter are assigned to this aggregation code.

12. The system according to any of the preceding claims,
which has an interface (28) to a higher-level system (30) in which the read codes (36) are verified and/or booked out.

13. A method for parallel mass reading of a plurality of codes (36) on a plurality of objects (34) arranged closely packed next to one another in a transport unit (32), wherein an image of the objects (34) is recorded with a camera unit (12) fastened to a frame, in which image code regions of the codes (36) are found and the code information of the codes (36) is read, and the image is displayed and the read codes (36) and/or objects (34) with read codes (36) are marked, wherein the transport unit (32) is placed on a support provided on the frame and unread codes (40) which could not be read from the image of the camera unit (12) are re-read with a hand-held reading unit (26) for reading codes (36, 40), and the code information read by the camera unit (12) and the hand-held reading unit (26) are collected together, wherein it is checked whether a re-read code (36, 40) has already been read before.

## Revendications

1. Système avec un châssis qui présente un support sur lequel est placée une unité de transport (32) avec une pluralité d'objets (34) disposés côte à côte de manière compacte, et avec un dispositif de lecture de code (10) pour une lecture de masse parallèle d'une pluralité de codes (36) sur la pluralité d'objets (34) disposés de manière compacte, dans lequel le dispositif de lecture de code (10) comprend une unité de caméra (12) fixée au châssis avec au moins une tête de caméra pour prendre une image des objets (34), une unité de commande et d'évaluation (18) configurée pour trouver dans l'image des régiones de code des codes (36) et pour lire l'informations de code des codes (36), ainsi qu'une unité d'affichage (20) pour afficher l'image et marquer les codes lus (36) et/ou les objets (34) avec des codes lus (36), dans lequel le dispositif de lecture de codes (10) comprend en outre une unité de lecture manuelle (26) pour lire les codes (36, 40) afin de relire des codes non lus (40) qui n'ont pas pu être lus à partir de l'image de l'unité de caméra (12), et de transmettre l'information de code relu à l'unité de commande et d'évaluation (18), l'unité de commande et d'évaluation (18) étant configurée pour vérifier si un code relu (36, 40) a déjà été lu auparavant.

2. Système selon la revendication 1,
dans lequel le dispositif de lecture de code (10) comprend plusieurs têtes de caméra, dans lequel l'unité de commande et d'évaluation (18) est configurée pour combiner les données d'image des têtes de caméra en une image commune et/ou pour lire les codes (36) dans les données d'image respectives et les comparer les unes aux autres.

3. Système selon la revendication 1 ou 2,
dans lequel les objets (34) sont des emballages ayant chacun un code unique (36), en particulier des emballages de médicaments ou de cigarettes.

4. Système selon l'une des revendications précédentes,
dans lequel l'unité de commande et d'évaluation (18) est configurée pour reconnaître et en particulier pour compter les objets (34) comme un rectangle respectif de bords détectés.

5. Système selon l'une des revendications précédentes,
dans lequel l'unité de commande et d'évaluation (18) est configurée pour marquer des codes (36) et/ou des objets (34) pour lesquels aucun code (36, 40) n'a encore été lu.

6. Système selon l'une des revendications précédentes,
dans lequel l'unité de commande et d'évaluation (18) est configurée pour localiser des codes de relecture (40, 44).

7. Système selon l'une des revendications précédentes,
dans lequel l'unité de commande et d'évaluation (18) est configurée pour localiser un spot lumineux de lecture généré par l'unité de lecture manuelle (26) sur les objets (34).

8. Système selon l'une des revendications précédentes,
dans lequel l'unité de commande et d'évaluation (18) est configurée pour marquer un code de relecture (40, 44).

9. Système selon l'une des revendications précédentes,
dans lequel l'unité d'affichage (20) est configurée comme un écran tactile via lequel le dispositif de lecture de code (10) est actionné.

10. Système selon l'une des revendications précédentes,
dans lequel l'unité de caméra (12) présente une unité d'éclairage variable (16a- b), en particulier avec plusieurs modules d'éclairage (16a- b), et dans lequel l'unité de commande et d'évaluation (18) est configurée pour enregistrer plusieurs fois les objets (34) sous différents éclairages pour la lecture parallèle des codes (36), en particulier avec un éclairage provenant de différentes directions.

11. Système selon l'une des revendications précédentes,
dans lequel l'unité de commande et d'évaluation (18) est configurée pour un mode d'agrégation dans lequel un code d'agrégation est d'abord lu et les codes lus (36) ensuite sont affectés à ce code d'agrégation.

12. Système selon l'une des revendications précédentes,
qui présente une interface (28) avec un système de niveau supérieur (30) dans lequel les codes lus (36) sont vérifiés et/ou biffées.

13. Procédé pour la lecture en masse parallèle d'une pluralité de codes (36) sur une pluralité d'objets (34) disposes côte à côte de manière compacte dans une unité de transport (32), dans lequel une image des objets (34) est enregistrée avec une unité de caméra (12) fixée sur un châssis, dans laquelle des régiones de code d'image des codes (36) sont trouvées et l'information de code des codes (36) est lue, et l'image est affichée et les codes lus (36) et/ou les objets (34) sont marqués avec des codes lus (36), dans lequel l'unité de transport (32) est placée sur un support prévu sur le châssis et les codes non lus (40) qui n'ont pas pu être lus à partir de l'image de l'unité de caméra (12) sont relus avec une unité de lecture manuelle (26) pour lire les codes (36, 40), et les informations de code lues par l'unité de caméra (12) et l'unité de lecture manuelle (26) sont collectées ensemble, dans lequel il est vérifié si un code relu (36, 40) a déjà été lu auparavant.
